# EUROPÄISCHE PATENTSCHRIFT

(11) **EP 2 734 127 B1**
(45) Veröffentlichungstag und Bekanntmachung des Hinweises auf die Patenterteilung: **31.05.2017**
(21) Anmeldenummer: 12738130.9
(22) Anmeldetag: 23.07.2012
(51) Int. Cl.: A61B 17/16

(54) **INSTRUMENTARIUM ZUM BEHANDELN VON SPINALKANALSTENOSEN**
INSTRUMENT SET FOR TREATING STENOSES OF THE SPINAL CANAL
INSTRUMENT CHIRURGICAL POUR LE TRAITEMENT DE STÉNOSES DU CANAL SPINAL

(30) Priorität: 22.07.2011 DE 202011103583 U
(43) Veröffentlichungstag der Anmeldung: 28.05.2014
(73) Patentinhaber: Fähndrich, Martin, 40547 Düsseldorf (DE); Alfen, Florian, 97082 Würzburg (DE)
(72) Erfinder: Fähndrich, Martin, 40547 Düsseldorf (DE); Alfen, Florian, 97082 Würzburg (DE)
(74) Vertreter: Schumacher & Willsau
(86) Internationale Anmeldenummer: PCT/EP2012/064438
(87) Internationale Veröffentlichungsnummer: WO 2013/014131

(56) Entgegenhaltungen:
- WO-A1-2011/060077
- US-A1- 2005 209 622
- US-A1- 2005 261 692
- US-A1- 2010 057 087

## Beschreibung

Die Erfindung betrifft ein Instrumentarium zum Behandeln von Spinalkanalstenosen.

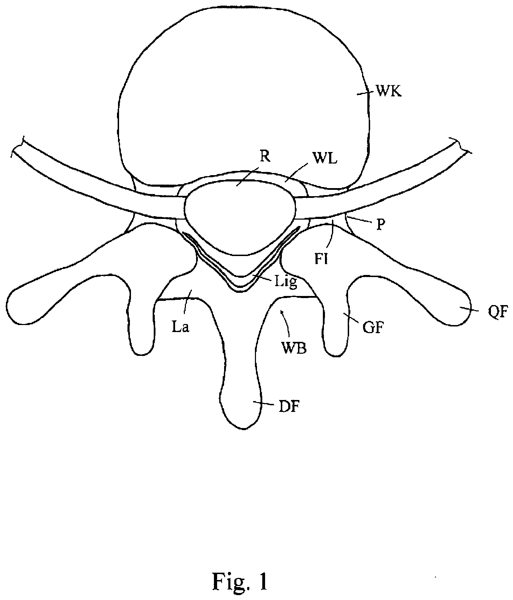

Im Inneren der aus einzelnen Wirbeln zusammengesetzten Wirbelsäule verläuft das Rückenmark. Fig. 1 zeigt einen Wirbel und das Rückenmark im Querschnitt. Die einzelnen Wirbel weisen jeweils einen massiven bauchwärtigen (zu dem menschlichen Bauch hin angeordneten) Teil, den Wirbelkörper (WK), und einen rückwärtigen Teil, den Wirbelbogen (WB), auf. Zwischen Wirbelkörper und Wirbelbogen befindet sich das Wirbelloch (WL). Die Wirbellöcher der Wirbel bilden den Spinalkanal, der das Rückenmark (R) aufnimmt, das auch als Dura bezeichnet wird. Um das Rückenmark in den Spinalkanal einzubetten, sind zwischen Wirbelkörper bzw. Wirbelbogen und Rückenmark Gewebebänder angeordnet. Diese Gewebebänder neigen mit zunehmendem Alter dazu, sich zu verhärten und gleichzeitig an Volumen zuzunehmen, was als Verknöcherung bezeichnet wird. Tritt eine solche Verknöcherung in größerem Umfang auf, kann es zu einer Einschnürung des Rückenmarks kommen, was Lähmungserscheinungen und Schmerzen hervorruft. Derartige Einschnürungen durch verknöchertes Gewebe werden Spinalkanalstenosen genannt.

Besonders häufig bilden sich Spinalkanalstenosen an einem rückwärtig (zu dem menschlichen Rücken hin) zwischen Wirbelbogen und Rückenmark angeordneten Gewebeband, dem gelben Band oder Ligamentum Flavum (Lig). Wie aus Fig. 1 ersichtlich ist, weist der Wirbelbogen in seinem bauchwärtigen Bereich zwei mit dem Wirbelkörper verbundene Ansätze, die Pedikel (P), und im rückwärtigen Bereich den hinteren Wirbelbogen auf, der auch als Lamina (La) bezeichnet wird. Von den beiden Übergangsstellen zwischen Pedikeln und Lamina ausgehend steht jeweils ein Querfortsatz (QF) in seitlicher Richtung der Wirbelsäule (lateral) vor. Von der Mitte des Wirbelbogens ausgehend erstreckt sich in rückwärtiger Richtung vorstehend der Dornfortsatz (DF). Zwischen dem Dornfortsatz und jedem der beiden Querfortsätze erstreckt sich ein Gelenkfortsatz (GF) in Wirbelsäulen-Längsrichtung nach oben und ein Gelenkfortsatz nach unten. Diese Gelenkfortsätze bilden im Zusammenwirken mit Gelenkfortsätzen des benachbarten Wirbels die Facettengelenke, über die die Wirbel relativ zueinander bewegt werden. Das Ligamentum Flavum befindet sich an der spinalkanalseitigen Oberfläche der Lamina einschließlich der Gelenkfortsätze.

Spinalkanalstenosen werden bisher durch operative Eingriffe behoben, bei denen der Eingriff im Zentrum des Rückens ansetzt, dort wo die Wirbelsäule verläuft. Dabei wird zunächst der betroffene Wirbelkörper weiträumig freigelegt, indem die Haut geöffnet und das die Wirbelsäule umgebende Muskelgewebe durchtrennt wird. Sodann wird aus dem Wirbelkörper derjenige Teil der Lamina, an dem sich die Spinalkanalstenosen gebildet haben, ausgestanzt. Diese Technik wird als Mikrolaminektomie bezeichnet und ist z. B. in "Preand postoperative complications of surgical treatment of lumbar spinal stenosis: prospective analysis of 306 patients" von Guigui, P., L. Cardinne, L. Rillardon, T Morais, A. Vuillemin, A, Deburge, Rev Chir Orthop Reparatrice Appar Mot. 88, 7: 669-677 (2002) beschrieben.

Bei diesem Eingriff muss häufig eine der beiden Hälften der Lamina (Hemilaminektomie) oder müssen sogar beide Hälften der Lamina (Laminektomie) vollständig entfernt werden, was zu einer erheblichen Destabilisierung des Wirbelkörpers führt. Die Wirbelsäule muss dann unter Umständen, insbesondere dann wenn mehrere Wirbelkörper betroffen sind, durch Verbindung mehrerer Wirbelkörper über Plattenelemente stabilisiert werden, die mittels Pedikelschrauben befestigt werden (Osteosynthese). Dies hat den Nachteil, dass die Wirbelsäule in dem entsprechenden Bereich ihre Beweglichkeit nahezu vollständig einbüßt. Weil in erheblichem Umfang Muskelgewebe durchtrennt werden muss und es bei dem Ausstanzen der Teile der Lamina zu einer starken Verwundung des umliegenden Gewebes kommt, ist die Mikrolaminektomie ferner mit den üblichen Nachteilen schwerer operativer Eingriffe verbunden. Dazu zählen Wundheilungsstörungen, Infektionsgefahr, lange Rekonvaleszenz, Vernarbung und Risiken der Anästhesie. Das bedeutet, dass sich ältere Menschen diesem Eingriff nicht ohne weiteres unterziehen können. Schließlich kann es zu gefährlichen, bis hin zu tödlichen Verletzungen kommen, wenn die Pedikelverschraubung aufgrund hoher Belastung ausbricht, z. B. wenn der Patient Sport ausübt und es zu nicht vorhersehbaren Bewegungen der Wirbelkörper kommt.

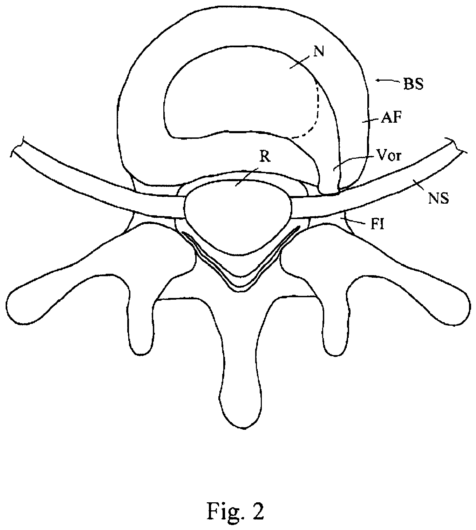

Im Zusammenhang mit einer anderen Erkrankung der Wirbelsäule, dem Bandscheibenvorfall, sind so genannte mikroinvasive Operationsmethoden bekannt. Die bei einem Bandscheibenvorfall auftretende Problematik ist in Fig. 2 dargestellt. Zwischen den einzelnen Wirbelkörpern befinden sich die Bandscheiben (BS), die als elastische Puffer zwischen den Wirbelkörpern dienen. Die Bandscheiben bestehen aus einem aus Bindegewebe und Knorpelgewebe bestehenden äußeren Ring, dem Annulus Fibrosus (AF), und einem gallertartigen inneren Kern, dem Nucleus (N). Der Nucleus kann sich verlagern oder durch Risse im Annulus Fibrosus (AF) austreten, was als Bandscheibenvorfall (Vor) bezeichnet wird. Oberhalb der Querfortsätze sind in den Wirbelkörpern seitliche Ausnehmungen vorhanden, die als Foramen Intervertebrale (FI) bezeichnet werden. Durch diese Ausnehmung verlaufen vom Rückenmark seitlich abzweigende Nervenstränge (NS), die zu einzelnen Organen, zu Bestandteilen des Muskelapparats etc. führen. Tritt eine größere Menge der gallertartigen Substanz des Nucleus aus, kann diese Masse auf den von dem Rückenmark seitlich abzweigenden Nervenstrang im Bereich der Wurzel Druck ausüben und heftige Schmerzen oder Lähmungserscheinungen auslösen.

Zur Behandlung dieser Beschwerden wurde die endoskopische transforaminale Nukleotomie entwickelt, die z. B. beschrieben ist in "Endoscopical foraminal removal of disc herniation" von T. Hoogland, J. Hallbauer, 4th International Spine Symposium, München September 1995. Vom seitlichen Rücken ausgehend wird ein mikroinvasiver Zugangsweg durch das Foramen Intervertebrale bis an die Bandscheibe gelegt. In diesen Zugangsweg wird ein Endoskop eingeführt. Durch den Arbeitskanal des Endoskops wird eine Mikrozange eingeführt, mit der das ausgetretene Nukleusgewebe, das den Bandscheibenvorfall bildet, entfernt wird, um die Beeinträchtigung des Nervenstrangs abzustellen.

Instrumente mit Abtrageeinrichtungen zum Gebrauch in der Wirbelsäulenchirurgie sind u.a. aus WO 2011/060077 A1, US 2005/0261692 A1, US 2005/0209622 A1 und US 2010/0057087 A1 bekannt, und die Präambel des Anspruchs 1 basiert auf der Offenbarung des letzten. Trotz größter Bemühungen in der Fachwelt ist es jedoch bisher nicht gelungen, mittels mikroinvasiver Methoden Spinalkanalstenosen erfolgreich zu beseitigen. Die für die transforaminale Nukleotomie entwickelten Zangen sind ungeeignet, um Spinalkanalstenosen abzutragen. Ferner finden sich Spinalkanalstenosen an Orten, die bislang ohne Wegstanzen von Teilen des Wirbelkörpers nicht zugänglich sind.

Der Erfindung liegt die Aufgabe zugrunde, die mikroinvasive Behandlung von Spinalkanalstenosen zu ermöglichen. Erfindungsgemäss wird diese Aufgabe gelöst mittels eines Instrumentariums zum mikroinvasiven Behandeln von Spinalkanalstenosen und/oder anderen Einengungen des Spinalkanals, aufweisend eine Abtrageeinrichtung mit einem Abtragekopf, der von einem mikroinvasiven Zugangsröhrchen, das ein in den Körper einführbares distales Ende aufweist, aufnehmbar ist und der an dem distalen Ende aus dem Zugangsröhrchen herausführbar und in mindestens eine Arbeitsposition bringbar ist; ein Abschirmelement zum Abschirmen des Abtragekopfes gegenüber der Dura, das von dem mikroinvasiven Zugangsröhrchen aufnehmbar ist und das an dem distalen Ende aus dem Zugangsröhrchen herausführbar und in mindestens eine Arbeitsstellung bringbar ist, in der das Abschirmelement das Zugangsröhrchen lateral überragt.

Weitere vorteilhafte Ausgestaltungen der Erfindung werden anhand von Ausführungsbeispielen erläutert, die überwiegend unter Bezug auf die Zeichnung beschrieben sind.
- Fig. 1: zeigt den Aufbau eines menschlichen Wirbelkörpers mit einem Beispiel für eine zu behandelnde Spinalkanalstenose;
- Fig. 2: zeigt die Problematik eines Bandscheibenvorfalls;
- Fig. 3A: zeigt ein Instrumentarium zum mikroinvasiven Abtragen von Spinalkanalstenosen in einem ersten Zustand;
- Fig. 3B: zeigt das Instrumentarium nach Fig. 3A in einem zweiten Zustand;
- Fig. 4A bis D: zeigen Schritte einer Anwendung des Instrumentariums zum mikroinvasiven Abtragen von Spinalkanalstenosen nach den Fig. 3A und B;
- Fig. 5: zeigt ein Instrumentarium zum mikroinvasiven Abtragen von Spinalkanalstenosen nach einem Ausführungsbeispiel der Erfindung;
- Fig. 6: zeigt einen Abtragekopf und ein Abschirmelement des Instrumentariums zum mikroinvasiven Abtragen von Spinalkanalstenosen und/oder anderen Einengungen des Spinalkanals nach einem Ausführungsbeispiel der Erfindung;
- Fig. 7: zeigt einen Abtragekopf und ein Abschirmelement des Instrumentariums zum mikroinvasiven Abtragen von Spinalkanalstenosen und/oder anderen Einengungen des Spinalkanals nach einem weiteren Ausführungsbeispiel der Erfindung;
- Fig. 8: zeigt einen Abtragekopf und ein Abschirmelement des Instrumentariums zum mikroinvasiven Abtragen von Spinalkanalstenosen und/oder anderen Einengungen des Spinalkanals nach noch einem weiteren Ausführungsbeispiel der Erfindung;
- Fig.9: zeigt ein Ausführungsbeispiel einer Betätigungsvorrichtung eines Abschirmelements des erfindungsgemäßen Instrumentariums zum mikroinvasiven Abtragen von Spinalkanalstenosen nach einem Ausführungsbeispiel der Erfindung.

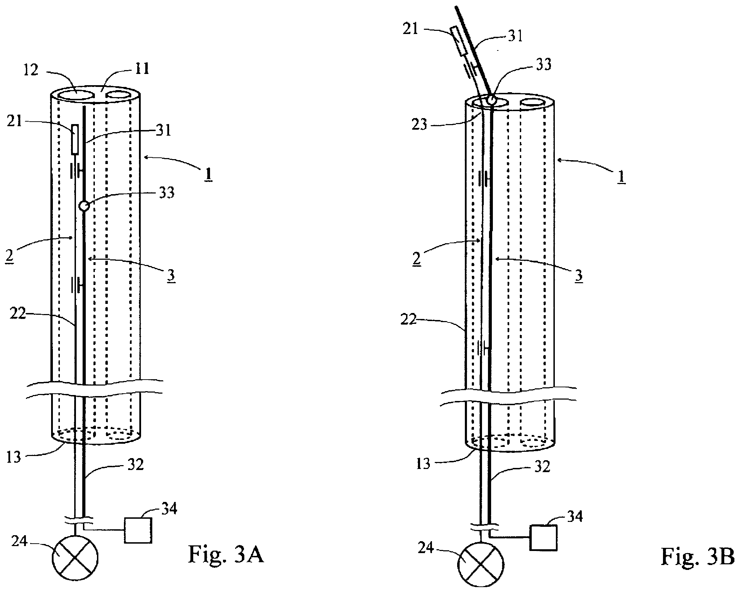

Die Fig. 3A und Fig. 3B zeigen ein Instrumentarium zum mikroinvasiven Abtragen von Spinalkanalstenosen und/oder anderen Einengungen des Spinalkanals. Das Instrumentarium weist eine Abtrageeinrichtung 2 und eine Abschirmeinrichtung 3 auf. Das Instrumentarium wird in Verbindung mit einem mikroinvasiven Zugangsröhrchen 1 mit einem in den Körper einführbaren distalen Ende 11 und einem zylinderförmigen ersten Arbeitskanal 12 eingesetzt, von dem die Abtrageeinrichtung 2 und die Abschirmeinrichtung 3 aufgenommen werden können. Das Zugangsröhrchen 1 kann eigens für das Zusammenwirken mit der Abtrageeinrichtung 2 und der Abschirmeinrichtung 3 konzipiert sein oder es kann sich um ein handelsübliches Röhrchen handeln. Das Zugangsröhrchen 1 kann Bestandteil des Instrumentariums zum mikroinvasiven Abtragen von Spinalkanalstenosen sein oder nicht.

Die Abtrageeinrichtung 2 weist einen Abtragekopf 21 und eine Welle 22 auf. Die Abschirmeinrichtung 3 weist ein Abschirmelement 31 und einen langgestreckten Halter 32 auf. Das Abschirmelement 31 ist an dem langgestreckten Halter 32 über ein Gelenk 33 abgestützt. An seinem proximalen, also dem Operateur zugewandten Ende weist die Abschirmeinrichtung 3 eine Betätigungsvorrichtung 34 auf, mit der bewirkt werden kann, dass das Abschirmelement 31 über das Gelenk 33 gegenüber dem langgestreckten Halter 32 abgeklappt wird. Die Welle 22 erstreckt sich entlang dem langgestreckten Halter 32 und dem Abschirmelement 31 und ist an ersterem oder letzterem oder beiden gelagert. An ihrem proximalen, also dem Operateur zugewandten Ende ist die Welle 22 an einen Antrieb 24 angeschlossen. Die Abtrageeinrichtung 2 ist an der Abschirmeinrichtung 3 so angebracht, dass der Abtragekopf 21 im Bereich des Abschirmelements 31 angeordnet ist. Dabei sind Abtragekopf 21 und Abschirmelement 31 so gestaltet und zueinander angeordnet, dass das Abschirmelement 31 den Abtragekopf 21 in erheblichen Bereichen gegenüber der Umgebung abschirmen kann.

Wie aus Fig. 3A ersichtlich ist, kann die Abtrageeinrichtung 2 eine Stellung einnehmen, in der die Welle 22 gerade ist und Welle 22 und Abtragekopf 21 fluchten. Die Abschirmeinrichtung 3 kann eine Stellung einnehmen, in der sich der langgestreckte Halter 32 und das Abschirmelement 31 im Wesentlichen entlang einer Geraden erstrecken und fluchten. Wenn die Abtrageeinrichtung 2 und die Abschirmeinrichtung 3 diese Stellungen einnehmen, können sie von dem proximalen Ende 13, d. h. von dem dem Operateur zugewandten Ende des Zugangsröhrchens 1, in den ersten Arbeitskanal 12 des Zugangsröhrchens 1 eingeschoben werden. Die Abtrageeinrichtung 2 und die Abschirmeinrichtung 3 sowie der erste Arbeitskanal 12 sind hinsichtlich ihrer Abmessungen so aufeinander abgestimmt, dass die Abtrageeinrichtung 2 und die Abschirmeinrichtung 3 im Wesentlichen vollständig von dem ersten Arbeitskanal 12 aufgenommen werden, wie dies in Fig. 3A dargestellt ist. Insbesondere werden der Abtragekopf 21 und das Abschirmelement 31 im Wesentlichen vollständig von dem ersten Arbeitskanal 12 aufgenommen.

Die Abtrageeinrichtung 2, die Abschirmeinrichtung 3 und das Zugangsröhrchen 1 sind so gestaltet, dass der Abtragekopf 21 bzw. das Abschirmelement 31 an dem distalen, also der Operationsstelle zugewandten Ende 11 des Zugangsröhrchens 1 aus diesem herausgeführt werden können. Dadurch kann der Abtragekopf 21 in unterschiedliche Arbeitspositionen gebracht werden. Auch das Abschirmelement 31 kann auf diese Weise in unterschiedliche Arbeitsstellungen gebracht werden. Eine erste Arbeitsposition des Abtragekopfes 21 und eine erste Arbeitsstellung des Abschirmelements 31 sind in Fig. 3B dargestellt. Der Abtragekopf 21 und das Abschirmelement 31 sind in der ersten Arbeitsposition bzw. in der ersten Arbeitsstellung gegenüber der Mittellinie des Zugangsröhrchens 1 geneigt und stehen gegenüber der gedachten Verlängerung des Zugangsröhrchens 1 seitlich (lateral) vor. Die Welle 22 nimmt in dieser Situation eine abgelenkte Form ein. Sie kann z. B. flexibel ausgebildet sein und in dieser Situation eine Biegung 23 beschreiben. Alternativ kann sie ein Gelenk aufweisen, z. B. ein Kardangelenk, und ist in dieser Situation abgeklappt. Das Abschirmelement 31 ist dann über das Gelenk 33 gegenüber dem langgestreckten Halter 32 abgeklappt. Der Abtragekopf 21 und das Abschirmelement 31 können weitere Arbeitspositionen bzw. Arbeitsstellungen einnehmen, in denen sie stärker oder weniger stark abgeklappt sind.

Bei der Abtrageeinrichtung 2 kann es sich um eine Fräseinrichtung handeln, die einen Fräskopf und eine Fräserwelle aufweist. Ebenso kann es sich z. B. um eine Hobeleinrichtung, eine Schleifeinrichtung, eine Schneideeinrichtung oder eine andere Einrichtung handeln, die zum Abtragen von Knochen- und/oder Gewebematerial geeignet ist.

Das Zugangsröhrchen 1 hat eine im Wesentlichen zylindrische Gestalt. Der in Längsrichtung verlaufende Arbeitskanal 12 weist einen im Wesentlichen gleich bleibenden Querschnitt auf. Der Arbeitskanal 12 ist so bemessen, dass die Abtrageeinrichtung 2 und die Abschirmeinrichtung 3, insbesondere der Abtragekopf 21 und das Abschirmelement 31, von dem proximalen Ende her in den Arbeitskanal 12 eingeschoben und an dem distalen Ende aus diesem herausgeführt werden können.

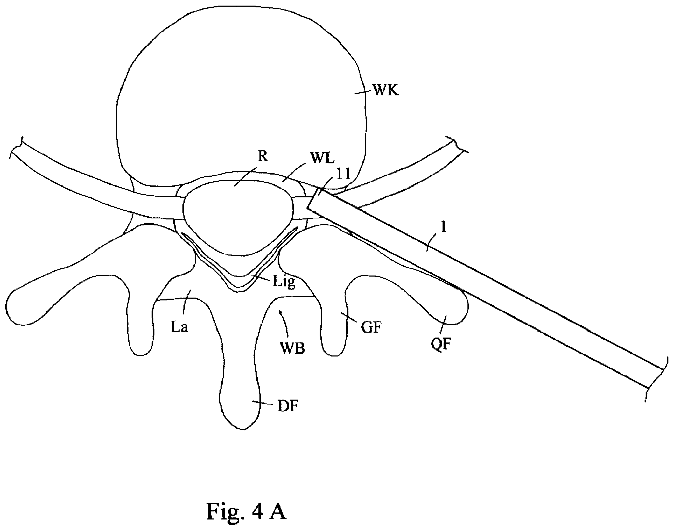

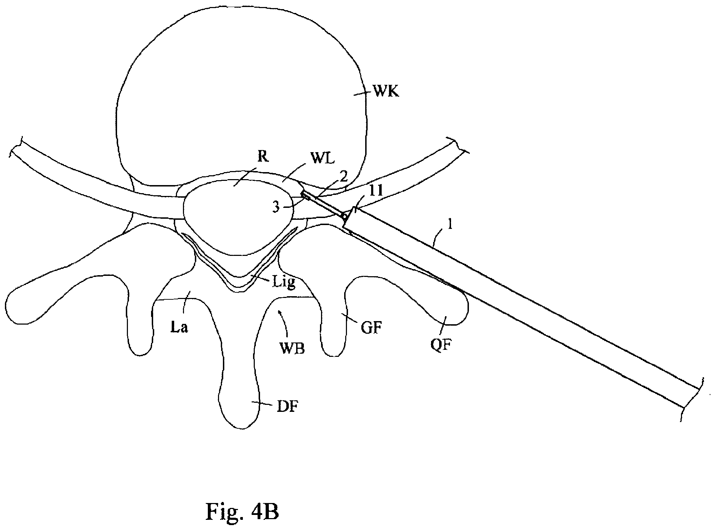

Die Fig. 4A bis D zeigen Schritte eines Beispiels für einen Operationsablauf, der mit dem Instrumentarium ermöglicht wird.
- Der Patient liegt auf der Seite. Von einer seitlichen Rückenpartie ausgehend wird ein Zugangsweg zum Foramen Intervertebrale gelegt, der geeignet ist, das Zugangsröhrchen 1 einzuführen.
- Das Zugangsröhrchen 1 wird über diesen Zugangsweg in das Foramen Intervertebrale eingeschoben. Bevorzugt wird das Zugangsröhrchen 1 so weit eingeschoben, dass es in den Bereich der Dura gelangt. Besonders bevorzugt wird das Zugangsröhrchen 1 so weit eingeschoben, bis dessen distales Ende 11 gegen den Wirbelkörper stößt, wie in Fig. 4A gezeigt ist.
- Das Zugangsröhrchen 1 wird sodann um eine definierte Strecke zurückgezogen. Diese Strecke kann z. B. so bemessen sein, dass sich das distale Ende 11 des Zugangsröhrchens 1 in einem Abstand von der Dura befindet, der der Länge des Abschirmelements 31 entspricht.
- Daraufhin wird das Abschirmelement 31 aus dem distalen Ende 11 des Zugangsröhrchens 1 heraus eine Strecke in Richtung der Dura vorgeschoben, so dass sich die in Fig. 4B dargestellte Position ergibt.
- Dann wird das Abschirmelement 31 durch Betätigen der Betätigungsvorrichtung 34 abgeklappt und auf diese Weise zur Rückenseite des Wirbels hin an der Dura vorbeigeführt.

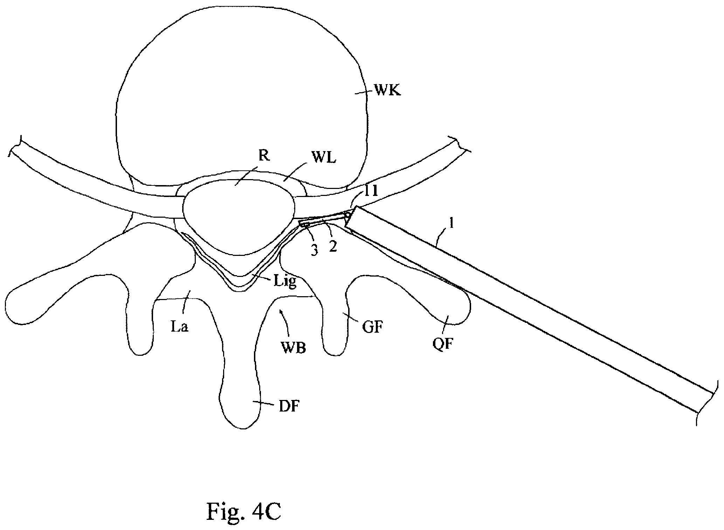

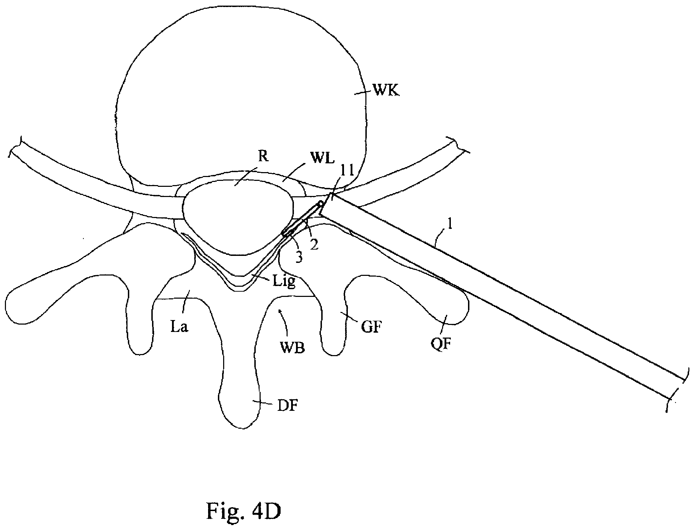

Das Abschirmelement 31 wird, wie in Fig. 4C gezeigt ist, so weit abgeklappt, dass das distale Ende des Abschirmelements 31 im Bereich zwischen der Dura und dem Wirbelbogen zu liegen kommt.
- Sobald das distale Ende des Abschirmelements 31 in diesem Bereich angeordnet ist, kann das Abschirmelement 31, wie in Fig. 4D dargestellt ist, weiter in den Zwischenraum zwischen der Dura und dem Wirbelbogen eingeführt werden, indem das Zugangsröhrchen 1 wieder etwas weiter in das Foramen Intervertebrale vorgeschoben und gleichzeitig das Abschirmelement 31 weiter abgeklappt wird. Die beiden Bewegungen können auch abwechselnd erfolgen. Bevorzugt erfolgen sie mehrmals abwechselnd.
- Sofern der Bewegung des Abschirmelements 31 zu dem Zwischenraum zwischen der Dura und dem Wirbelbogen hin bzw. in diesen Zwischenraum hinein Verknöcherungen oder andere verhärtete Strukturen im Wege stehen, können diese mittels der Abtrageeinrichtung 2 abgetragen werden.

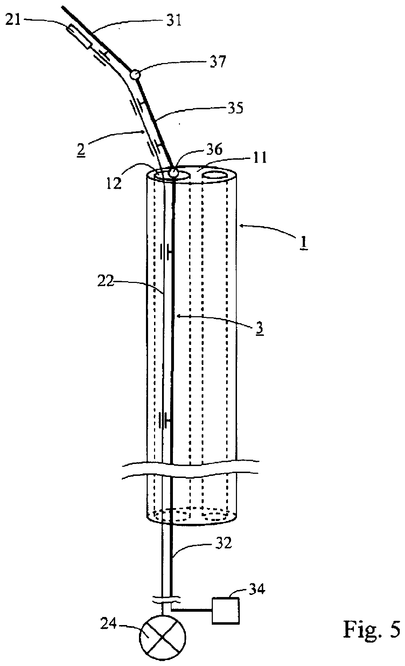

Fig. 5 zeigt ein Instrumentarium zum mikroinvasiven Abtragen von Spinalkanalstenosen und/oder anderen Einengungen des Spinalkanals nach einem Ausführungsbeispiel der Erfindung. Das Instrumentarium gemäß diesem Ausführungsbeispiel unterscheidet sich von dem Instrumentarium nach den Fig. 3A und Fig. 3B dadurch, dass zwischen dem langgestreckten Halter 32 und dem Abschirmelement 31 ein Zwischenelement 35 vorgesehen ist. Über ein Gelenk 36 ist das Zwischenelement 35 gegenüber dem langgestreckten Halter 32 abklappbar. An dem Zwischenelement 35 ist das Abschirmelement 31 über ein Gelenk 37 abklappbar angeordnet. An seinem proximalen, also dem Operateur zugewandten Ende weist das Abschirmelement 3 eine Betätigungsvorrichtung 34 auf, mit der bewirkt werden kann, dass das Abschirmelement 31 über das Gelenk 37 gegenüber dem Zwischenelement 35 abgeklappt wird, und bewirkt werden kann, dass das Zwischenelement 35 über das Gelenk 36 gegenüber dem langgestreckten Halter 32 abgeklappt wird. In der Fig. 5 ist das Instrumentarium gemäß diesem Ausführungsbeispiel in einem Zustand dargestellt, der dem in Fig. 3B dargestellten Zustand des zuvor beschriebenen Ausführungsbeispiels entspricht. Das heißt, der Abtragekopf 21 befindet sich in einer ersten Arbeitsposition und das Abschirmelement 31 befindet sich in einer ersten Arbeitsstellung.

Ein Beispiel für einen Operationsablauf, der mit dem Instrumentarium nach diesem Ausführungsbeispiel der Erfindung ermöglicht wird, kann wie folgt beschrieben werden:
- Zunächst erfolgt ein Ablauf entsprechend der Beschreibung oben unter Bezugnahme auf die Fig. 4A bis Fig. 4D, jedoch mit der Maßgabe, dass das Abschirmelement 31 einerseits gegenüber dem langgestreckten Halter 32 und dem Zwischenelement 35 andererseits abgeklappt wird und der langgestreckte Halter 32 und das Zwischenelement 35 zueinander fluchtend verbleiben.
- Wenn das Abschirmelement 31 auf diese Weise in den Zwischenraum zwischen der Dura und dem Wirbelbogen eingeführt ist, wird das Zugangsröhrchen 1 erneut um eine definierte Strecke zurückgezogen. Diese Strecke kann z. B. so bemessen sein, dass sie der Länge des Zwischenelements 35 entspricht, so dass gerade das Gelenk 36 aus dem Zugangsröhrchen 1 vorsteht.
- Dann wird das Zwischenelement 35 durch Betätigen der Betätigungsvorrichtung 34 abgeklappt und auf diese Weise zur Rückenseite des Wirbels hin an der Dura vorbeigeführt. Gleichzeitig kann die Abklappstellung des Abschirmelements 31 gegenüber dem Zwischenelement 35 über die Betätigungsvorrichtung 34 verändert werden. Typischerweise wird der Abklappwinkel vermindert. Durch das Zusammenspiel der beiden Klappbewegungen wird das Abschirmelement 31 weiter in die Lücke zwischen Dura und Wirbelbogen eingeschoben. Dabei kann das Zwischenelement 35 so weit abgeklappt werden, dass auch das distale Ende des Zwischenelements 35 im Bereich zwischen der Dura und dem Wirbelbogen zu liegen kommt.
- Sobald das distale Ende des Zwischenelements 35 in dem Bereich zwischen der Dura und dem Wirbelbogen angeordnet ist, können das Abschirmelement 31 und das Zwischenelement 35 weiter in den Zwischenraum zwischen der Dura und dem Wirbelbogen eingeführt werden, indem das Zugangsröhrchen 1 wieder etwas weiter in das Foramen Intervertebrale vorgeschoben und gleichzeitig das Zwischenelement 35 weiter abgeklappt wird. Währenddessen wird gegebenenfalls der Abklappwinkel zwischen dem Zwischenelement 35 und dem Abschirmelement 31 weiter vermindert. Die Bewegungen des Einschiebens des Zugangsröhrchens 1 und des Abklappens des Zwischenelements 35 und des Veränderns des Abklappwinkels zwischen dem Zwischenelement 35 und dem Abschirmelement 31 können auch abwechselnd erfolgen. Bevorzugt erfolgen sie mehrmals abwechselnd.
- Soweit der Bewegung des Abschirmelements 31 weiter hinein in den Zwischenraum zwischen der Dura und dem Wirbelbogen hin bzw. in diesen Zwischenraum hinein Verknöcherungen oder andere verhärtete Strukturen im Wege stehen, werden diese mittels der Abtrageeinrichtung 2 abgetragen.

Weitere (in den Figuren nicht dargestellte) Ausführungsbeispiele des erfindungsgemäßen Instrumentariums werden gebildet, indem zusätzlich zu dem Zwischenelement 35 ein weiteres Zwischenstück oder mehrere weitere Zwischenstücke vorgesehen sind, die jeweils über Gelenke angelenkt sind und die Beweglichkeit zwischen dem Abschirmelement 31 und dem langgestreckten Halter 32 weiter vergrößern. Das Einführen des Abschirmelements 31 in den Zwischenraum zwischen Dura und Ligamentum Flavum erfolgt nach demselben Prinzip wie bei den zuvor beschriebenen Ausführungsbeispielen.

Weitere (in den Figuren nicht dargestellte) Ausführungsbeispiele des erfindungsgemäßen Instrumentariums werden gebildet, indem das Zugangsröhrchen 1 anders als bei den unter Bezug auf die Fig. 3 und Fig. 5 beschriebenen Ausführungsbeispielen ausgestaltet wird. Bei einer bevorzugten Ausführungsform weist der in dem Zugangsröhrchen 1 vorgesehene erste Arbeitskanal 12 einen vom Operateur abgewandten distalen Abschnitt, in dem der erste Arbeitskanal 12 weiter ist, um den Abtragekopf 21 und/oder das Abschirmelement 31 aufzunehmen, und einen proximalen Abschnitt auf, in dem der erste Arbeitskanal 12 enger ist. Bei einer anderen bevorzugten Ausführungsform weist der erste Arbeitskanal 12 eine distale Öffnung auf, die sich sowohl über einen Teil der Stirnseite des Zugangsröhrchens 1 als auch über einen Teil der Mantelfläche des Zugangsröhrchens 1 erstreckt. Bevorzugt ist am distalen Ende des Zugangsröhrchens in der Mantelfläche an einer Seite ein sich axial erstreckender Schlitz gebildet. Diese sich auch auf die Mantelfläche erstreckende Öffnung dient dazu, das Abschirmelement 31 und den Abtragekopf 21 bereits abklappen zu können, bevor die Abschirmeinrichtung 3 so weit vorgeschoben ist, dass das Gelenk 33, das Gelenk 36 und/oder das Gelenk 37 aus dem Zugangsröhrchen 1 heraussteht. Weiter bevorzugt werden die Gestaltungen mit einem weiteren und einem engeren Abschnitt des ersten Arbeitskanals 12 und mit einer sich auch auf die Mantelfläche erstreckenden Öffnung kombiniert.

Das Instrumentarium zum mikroinvasiven Abtragen von Spinalkanalstenosen und/oder anderen Einengungen des Spinalkanals kann eine endoskopische Optik (in den Figuren nicht gezeigt) aufweisen. Mit dieser Optik ist es möglich, den Operationsablauf unter Sicht durchzuführen. Um die Optik in eine Position zu bringen, in der sie eine Bildgebung des Arbeitsbereichs des Abtragekopfs 21 und des Bereichs ermöglicht, in dem die Abklappbewegungen des Abschirmelements 31 stattfinden, kann das Zugangsröhrchen 1 einen zweiten Arbeitskanal aufweisen, durch den die endoskopische Optik hindurchgeführt wird. Dadurch kann insbesondere Sicht auf die Dura und die das Foramen Intervertebrale durchtretenden Nervenbahnen ermöglicht werden. Dadurch kann sichergestellt werden, dass die Bewegungen des Abtragekopfs 21 und des Abschirmelements 31 so geführt werden, dass Dura und Nervenbahnen nicht verletzt werden.

Das Zugangsröhrchen 1 kann dann insgesamt vier oder mehr Kanäle aufweisen, z. B. den ersten Arbeitskanal 12, den zweiten Arbeitskanal und zwei Spülkanäle und ggf. einen oder mehrere weitere Kanäle für andere Funktionen.

Bevorzugt ist das Zugangsröhrchen 1 an seiner Stirnseite von einer Schnittfläche abgeschlossen, die mit einer zur Längsachse des Zugangsröhrchens 1 senkrechten Ebene einen Winkel von mindestens 20°, weiter bevorzugt mindestens 40°, bildet. Bevorzugt handelt es sich um eine elliptische Schnittfläche wie sie bei einem Schrägschnitt eines Zylinderrohrs entsteht. Bevorzugt endet der zum Durchführen der Optik vorgesehene zweite Arbeitskanal im Bereich des Endes der elliptischen Schnittfläche in Längsrichtung, das in Richtung der Achse des Zugangsröhrchens 1 am weitesten vorsteht, d. h. dem vorstehenden Ellipsenende. Der erste Arbeitskanal 12 endet im Bereich des entgegengesetzten Endes der elliptischen Schnittfläche, d. h. dem abfallenden Ellipsenende. Dabei können der erste Arbeitskanal 12 und der zweite Arbeitskanal in Querrichtung der elliptischen Schnittfläche versetzt angeordnet sein, z. B. der erste Arbeitskanal 12 mit seinem Mittelpunkt links von der längeren Achse der elliptischen Schnittfläche, der zweite Arbeitskanal mit seinem Mittelpunkt rechts von der längeren Achse. Dadurch wird erreicht, dass gleichzeitig eine gute Sicht auf den Arbeitsbereich der Abtrageeinrichtung 2, die Dura und die Nervenbahnen sichergestellt wird und vermieden wird, dass die Optik die Bewegungsabläufe beim Positionieren des Abtragekopfs 21 und des Abschirmelements 31 behindert.

Bei einer anderen (nicht in den Figuren gezeigten) Ausführungsform weist das erfindungsgemäße Instrumentarium zum mikroinvasiven Abtragen von Spinalkanalstenosen ein Zugangsröhrchen 1 auf, bei dem der erste Arbeitskanal 12 zum Aufnehmen der Abtrageeinrichtung 2 eingerichtet ist und ein dritter von dem ersten verschiedener Arbeitskanal (in den Figuren nicht gezeigt) vorgesehen ist, der zum Aufnehmen der Abschirmeinrichtung 3 vorgesehen ist. Die Welle 22 kann dabei durch Lagerstellen gelagert sein, die in dem ersten Arbeitskanal 12 vorgesehen sind. Der langgestreckte Halter 32 kann durch Lagerstellen gelagert sein, die in dem dritten Arbeitskanal vorgesehen sind.

Das Zugangsröhrchen 1 kann dann insgesamt fünf oder mehr Kanäle aufweisen, z. B. den ersten Arbeitskanal 12, den zweiten Arbeitskanal zum Durchführen einer Optik, den dritten Arbeitskanal, einen oder zwei Spülkanäle und ggf. einen oder mehrere weitere Kanäle für andere Funktionen.

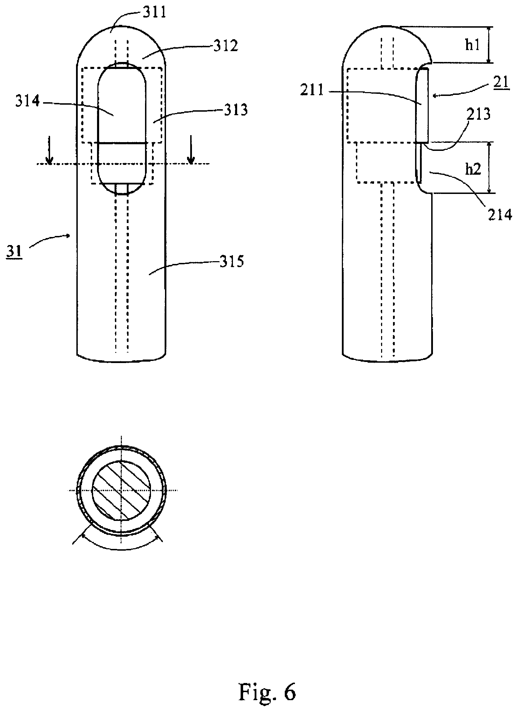

Nachfolgend werden unter Bezug auf die Fig. 6 der Abtragekopf 21 und das Abschirmelement 31 des Instrumentariums zum mikroinvasiven Abtragen von Spinalkanalstenosen und/oder anderen Einengungen des Spinalkanals nach einem Ausführungsbeispiel der Erfindung beschrieben. In dem in Fig. 6 gezeigten Ausführungsbeispiel ist die Abtrageeinrichtung 2 eine Fräseinrichtung und der Abtragekopf 21 ein Fräserkopf. Das Abschirmelement 31 ist so ausgebildet, dass es weite Teile des Abtragekopfs 21 abschirmt. Das Abschirmelement 31 weist an dem distalen Ende einen Stirnteil 311 auf, der den gesamten radial/azimutalen Querschnitt des Abtragekopfs 21 abdeckt. Ferner weist das Abschirmelement 31 bevorzugt einen oberen Mantelteil 312 auf, der den Abtragekopf 21 und ggf. dessen Lagerung über den gesamten Umfang abdeckt. Dann weist das Abschirmelement 31 einen mittleren Mantelteil 313 auf, der den Abtragekopf 21 über einen Teil des Umfangs abdeckt. In dem mittleren Mantelteil 313 ist ein Fenster 314 vorgesehen, das sich über einen Teil des Umfangs erstreckt und hinter dem der Abtragekopf 21 freiliegt. Schließlich weist das Abschirmelement 31 bevorzugt einen unteren Mantelteil 315 auf, der den Abtragekopf 21 und ggf. dessen Lagerung wiederum über den gesamten Umfang abdeckt. Die so beschriebene Geometrie hat den Vorteil, dass beim Vordringen der Abtrageeinrichtung 2 und der Abschirmeinrichtung 3 in den Zwischenraum zwischen der Dura und dem Ligamentum Flavum, das in den Fig. 4C und Fig. 4D illustriert ist, der Abtragekopf 21 in den beim Vordringen vorne befindlichen Bereichen durch den Stirnteil 311 und ggf. den oberen Mantelteil 312 hinreichend abgeschirmt ist, um die Dura zu schützen. Andererseits folgt bereits in relativ kurzem Abstand hinter der Spitze des Abschirmelements 31 mit dem Fenster 314 der Bereich, in dem der Abtragekopf 21 wirksam wird und Material abtragen kann. Bevorzugt beträgt der Abstand h1 zwischen dem distalen Ende der Abschirmeinrichtung 3 und dem oberen Ende des wirksamen Teils des Abtragekopfs 21 mindestens 2 mm, bevorzugt mindestens 4 mm, weiter bevorzugt mindestens 6 mm. Das Abschirmelement 31 kann einstückig oder mehrstückig ausgebildet sein. Gleiches gilt für jedes einzelne der hier beschriebenen Bestandteile der Abtrageeinrichtung 2 und der Abschirmeinrichtung 3.

In dem Ausführungsbeispiel nach Fig. 6 weist der Abtragekopf 21 einen Fräserabschnitt 211 mit zylinderförmiger Grundform auf. Der Fräserabschnitt 211 ist zu dem proximalen Ende hin (dem näher an der Welle 22 angeordneten Ende) durch einen proximalen Fräserabsatz 213 begrenzt. Bevorzugt verringert sich an diesem proximalen Fräserabsatz 213 der Durchmesser des Abtragekopfs 21 um mindestens 0,5 mm, bevorzugt um mindestens 1 mm, weiter bevorzugt um mindestens 2 mm. Auf den proximalen Fräserabsatz 213 folgt ein proximaler Freiraumabschnitt 214, in dem die Abtrageeinrichtung 2 gegenüber dem Fräserabschnitt 211 mindestens um die genannten Werte zurücktritt, und gleichzeitig in dem Fenster 314 freiliegt. Dieser Freiraumabschnitt 214 weist eine Länge h2 von mindestens 2 mm, bevorzugt von mindestens 4 mm, weiter bevorzugt von mindestens 6 mm auf.

Die so beschriebene Geometrie hat den Vorteil, dass die Gefahr einer Verletzung der Dura verringert, gleichzeitig aber ein besonders wirksames Abtragen von Stenosen sowie anderem Knochen- und Gewebematerial ermöglicht wird. Die Abschirmeinrichtung 3 und die Abtrageeinrichtung 2 werden, z. B. mittels der oben unter Bezug auf die Fig. 4A bis D beschriebenen Technik, in den Zwischenraum zwischen der Dura und dem Ligamentum Flavum eingeführt. Dabei wird der Abtragekopf 21 an einem abzutragenden Bereich zunächst vorbeigeführt. Der Antrieb der Abtrageeinrichtung 2 kann dabei bereits eingeschaltet sein oder nicht. Sodann wird der Abtragekopf 21 bei eingeschaltetem Antrieb in dem Zwischenraum zwischen der Dura und dem Ligamentum Flavum wieder in Richtung des Foramen Intervertebrale zurückgezogen. Gleichzeitig kann der Abtragekopf 21 durch Betätigung der Betätigungsvorrichtung 34 des Abschirmelements 31 gegen das Ligamentum Flavum bzw. in dessen Bereich vorhandene Stenosen oder gegen sonstige Knochen- oder Gewebestrukturen gedrückt werden. Dadurch kommt es zu einem besonders wirkungsvollen Abtragen von Material, insbesondere an dem proximalen Fräserabsatz 213, aber auch an der Umfangsfläche des Fräserabschnitts 211. Durch den proximalen Freiraumabschnitt 214 wird gefördert, dass der proximale Fräserabsatz 213 mit den abzutragenden Strukturen gut in Kontakt kommt, weil vorstehende Strukturen in den proximalen Freiraumabschnitt 214 hineinragen können. Dadurch dass das Abtragen durch eine Rückwärtsbewegung erfolgt und an einer von der Dura abgewandten Seite des Abtragekopfs 21 erfolgt, wird gefördert, dass es bei richtiger Handhabung nicht zu Verletzungen der Dura kommt.

Das Abschirmelement 31 hat bei dieser Ausführungsform einen kreisförmigen Querschnitt. Der Öffnungswinkel des Fensters 314 beträgt zwischen 60° und 180°, bevorzugt zwischen 80° und 150°, weiter bevorzugt zwischen 100° und 120°. Dadurch wird gefördert, dass es bei richtiger Handhabung nicht zu Verletzungen der Dura kommen kann und gleichzeitig ein effektives Abtragen erreicht wird.

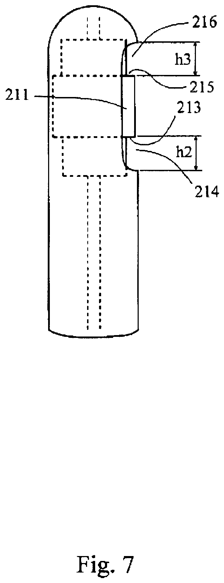

Fig. 7 zeigt ein weiteres Ausführungsbeispiel für die Abtrageeinrichtung 2 und das Abschirmelement 31. Bei dem in Fig. 7 gezeigten Ausführungsbeispiel ist die Abtrageeinrichtung 2 ebenfalls eine Fräseinrichtung und der Abtragekopf 21 ein Fräserkopf. Das Abschirmelement 31 ist ganz überwiegend genauso aufgebaut wie bei dem unter Bezug auf Fig. 6 beschriebenen Ausführungsbeispiel. Auch der Abtragekopf 21 ist ähnlich aufgebaut. Er weist ebenfalls einen Fräserabschnitt 211 mit zylinderförmiger Grundform auf. Im Unterschied zu dem Ausführungsbeispiel nach Fig. 6 ist bei dem Ausführungsbeispiel nach Fig. 7 der Fräserabschnitt 211 zu dem distalen Ende hin durch einen distalen Fräserabsatz 215 begrenzt. Dieser distale Fräserabsatz 215 kann bevorzugt alternativ und besonders bevorzugt zusätzlich zu dem proximalen Fräserabsatz 215 vorgesehen sein. Bevorzugt verringert sich an dem distalen Fräserabsatz 215 der Durchmesser des Abtragekopfs 21 um mindestens 0,5 mm, weiter bevorzugt um mindestens 1 mm, noch weiter bevorzugt um mindestens 2 mm. Auf den distalen Fräserabsatz 215 folgt zu dem distalen Ende hin ein distaler Freiraumabschnitt 216, in dem der Abtragekopf 21 gegenüber dem Fräserabschnitt 211 mindestens um die genannten Werte zurücktritt, und gleichzeitig in dem Fenster 314 freiliegt. Dieser distale Freiraumabschnitt 216 weist eine Länge h3 von mindestens 2 mm, bevorzugt von mindestens 4 mm, weiter bevorzugt von mindestens 6 mm auf.

Mit dem Abtragekopf 21 und dem Abschirmelement 31 nach diesem Ausführungsbeispiel wird ein besonders effektives Abtragen bereits bei deren Vorwärtsbewegung hinein in den Zwischenraum zwischen der Dura und dem Ligamentum Flavum ermöglicht. Dies hat den Vorteil, dass die Gefahr gering gehalten wird, dass ein Hindernis, z. B. in Gestalt einer Stenose, ein weiteres Vorrücken des Abschirmelements 31 und des Abtragekopfs 21 vereitelt. Steht ein solches Hindernis im Weg, so müssen das Abschirmelement 31 und der Abtragekopf 21 nur um eine geringe Strecke an dem Hindernis vorbei gezwängt werden, bis das Hindernis mittels des Abtragekopfes 21 bearbeitet bzw. abgetragen werden kann. Die Abschirmeinrichtung 3 und die Abtrageeinrichtung 2 mit dem Abschirmelement 31 bzw. dem Abtragekopf 21 werden, z. B. mittels der oben unter Bezug auf die Fig. 4A bis D beschriebenen Technik, in den Zwischenraum zwischen der Dura und dem Ligamentum Flavum vorgeschoben. Der Antrieb der Abtrageeinrichtung 2 kann dabei bereits eingeschaltet sein oder nicht. Gleichzeitig kann der Abtragekopf 21 durch Betätigung der Betätigungsvorrichtung 34 des Abschirmelements 31 gegen das Ligamentum Flavum bzw. in dessen Bereich vorhandene Stenosen oder gegen sonstige Knochen- oder Gewebestrukturen gedrückt werden. Dadurch kommt es zu einem besonders wirkungsvollen Abtragen von Material, insbesondere an dem distalen Fräserabsatz 215, aber auch an der Umfangsfläche des Fräserabschnitts 211. Durch den oberen Freiraumabschnitt 216 wird gefördert, dass der distale Fräserabsatz 215 mit den abzutragenden Strukturen gut in Kontakt kommt, weil vorstehende Strukturen in den distalen Freiraumabschnitt 216 hineinragen können.

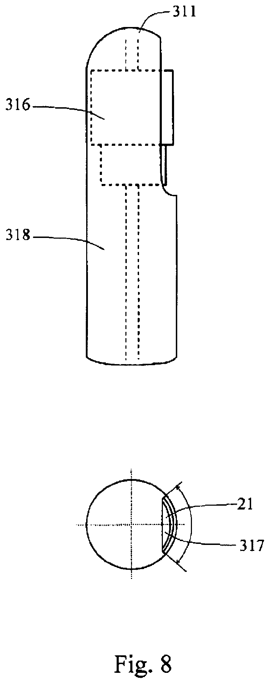

Fig. 8 zeigt noch ein anderes Ausführungsbeispiel für den Abtragekopf 21 und das Abschirmelement 31. Bei dem in Fig. 8 gezeigten Ausführungsbeispiel ist die Abtrageeinrichtung 2 ebenfalls eine Fräseinrichtung und der Abtragekopf 21 ein Fräserkopf. Das Abschirmelement 31 ist ebenso wie bei den Ausführungsbeispielen nach den Fig. 6 und Fig. 7 so ausgebildet, dass es weite Teile der Abtrageeinrichtung 2 abschirmt. Ferner weist das Abschirmelement 31 auch an dem distalen Ende einen Stirnteil 311 auf. Anders als bei den Ausführungsbeispielen nach den Fig. 6 und Fig. 7 deckt der Stirnteil 311 bei dem Ausführungsbeispiel nach Fig. 8 jedoch nicht den gesamten radial/azimutalen Querschnitt des Abtragekopfs 21 ab. Vielmehr liegt zur Stirnseite hin ein Teil des Abtragekopfs 21 frei. In dem distalen Mantelteil 316, der sich an die Stirnseite anschließt, ist eine Ausnehmung 317 vorgesehen, die sich über einen Teil des Umfangs erstreckt und hinter der der Abtragekopf 21 freiliegt. Weiter kann sich ein proximaler Mantelteil 318 anschließen, der den Abtragekopf 21 und ggf. dessen Lagerung über den gesamten Umfang abdeckt. Der Winkelbereich, über den sich die Ausnehmung 317 erstreckt, beträgt zwischen 60° und 180°, bevorzugt zwischen 80° und 150°, weiter bevorzugt zwischen 100° und 120°.

Die so beschriebene Geometrie hat den Vorteil, dass praktisch unmittelbar an der Spitze des Abschirmelements 31 der Abtragekopf 21 freiliegt und wirksam Material abtragen kann. Dies hat den Vorteil, dass die Gefahr gering gehalten wird, dass ein Hindernis, z. B. in Gestalt einer Stenose, ein weiteres Vorrücken des Abschirmelements 31 und des Abtragekopfs 21 vereitelt. Steht ein solches Hindernis im Weg, so kommt dieses mit dem an der Stirnseite der Abschirmeinrichtung 3 teilweise freiliegenden Abtragekopf 21 in Berührung und wird dadurch abgetragen, bis es einem weiteren Vorrücken nicht mehr im Wege steht. Gleichzeitig deckt der distale Mantelteil 316 den Abtragekopf 21 über den größeren Teil des Umfangs ab und verhindert in diesem Bereich, dass der Abtragekopf 21 die Dura verletzen kann.

Bei weiteren erfindungsgemäßen Ausführungsformen weist das Abschirmelement 31 mindestens eine Wand auf, die den Abtragekopf 21 zu der Seite der Dura hin abschirmt, und weist bevorzugt an seinen vom proximalen Ende zum distalen Ende verlaufenden seitlichen Flanken einen Abschnitt auf, der den Abtragekopf 21 seitlich abschirmt. Gleichzeitig oder davon unabhängig ist es bevorzugt, dass das Abschirmelement 31 von seinem proximalen Ende zum distalen Ende einen gekrümmten Verlauf aufweist. Weiter bevorzugt weist das Abschirmelement 31 von seinem proximalen Ende zum distalen Ende einen S-förmig gekrümmten Verlauf derart auf, dass das distale Ende in Abklapprichtung gekrümmt ist und sich zu dem proximalen Ende hin ein entgegengesetzt gekrümmter längerer Bogen anschließt. Bevorzugt umgibt das Abschirmelement 31 den Abtragekopf 21 weitgehend und ist an der Seite, zu der hin das Abschirmelement 31 bewegt wird, wenn es aus dem Zugangsröhrchen 1 herausgeführt und in die Arbeitsstellung gebracht wird, mit einer fensterartigen Aussparung versehen, in der der Abtragekopf 21 freiliegt.

Bevorzugt ist der Abtragekopf 21 bei den in den Fig. 6 bis Fig. 8 dargestellten Ausführungsformen, aber auch bei anderen Ausführungsformen, in seiner Position an dem Abschirmelement 31 verstellbar. Insbesondere kann eine Verstellbarkeit in Längsrichtung des Abschirmelements 31 vorgesehen sein. Dabei ist mit Längsrichtung die Ausdehnung von dem proximalen Ende des Abschirmelements 31 zu dem distalen Ende des Abschirmelements 31 gemeint. Ebenso oder zusätzlich kann eine Verstellbarkeit in Querrichtung des Abschirmelements 31 vorgesehen sein. Bevorzugt ist der Abtragekopf 21 über den distalen Rand des Abschirmelements 31 hinaus und/oder über einen seitlichen Rand oder die seitlichen Ränder des Abschirmelements 31 hinaus verstellbar. Besonders bevorzugt ist der Abtragekopf 21 über die gesamte Länge des Abschirmelements 31 verstellbar. Ferner wird bevorzugt, wenn der Abtragekopf 21 in seiner Orientierung verstellbar ist.

Bevorzugt weist das erfindungsgemäße Instrumentarium mehrere unterschiedliche Abtrageeinrichtungen 2 auf, die je nach Bedarf für bestimmte Abtrageaufgaben eingesetzt werden.

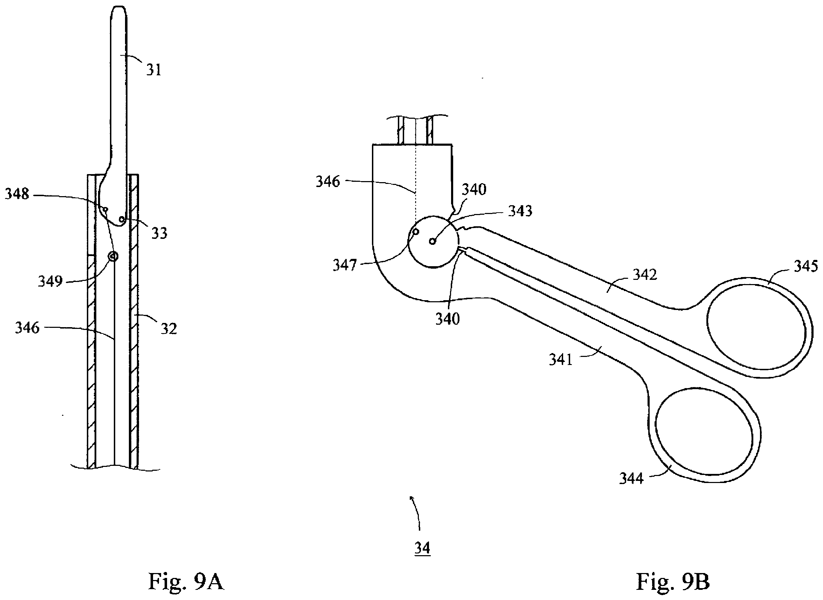

Fig. 9A und B zeigen ein Ausführungsbeispiel einer Betätigungsvorrichtung 34 eines Abschirmelements 31 eines erfindungsgemäßen Instrumentariums zum mikroinvasiven Abtragen von Spinalkanalstenosen. Wie aus Fig. 9A ersichtlich ist, ist bei diesem Ausführungsbeispiel der langgestreckte Halter 32 hohl ausgebildet. Im distalen Endbereich des langgestreckten Halters 32 ist das proximale Ende des Abschirmelements 31 aufgenommen. Das Gelenk 33 ist mit einem Bolzen und einer Gleitlagerung ausgeführt. Der Bolzen ist an dem Abschirmelement 31 fest und drehbar in Lagerbohrungen in dem langgestreckten Halter 32 aufgenommen oder an dem langgestreckten Halter 32 fest und drehbar in Lagerbohrungen in dem Abschirmelement 31 aufgenommen. Das Gelenk 33 ist an der einen Seite des hohl ausgebildeten langgestreckten Halters 32 vorgesehen. An der gegenüberliegenden Seite des langgestreckten Halters 32 ist eine Anlenkeinrichtung in Form eines weiteren Bolzens 348 vorgesehen, an dem ein Stelldraht 346 der Betätigungsvorrichtung 34 angreift. Der Stelldraht 346 wird über eine Umlenkeinrichtung 349 umgelenkt und erstreckt sich im Übrigen im Hohlraum des hohl ausgebildeten langgestreckten Halters 32 in dessen Längsrichtung. Durch Ziehen an dem Stelldraht 346 wird das Abschirmelement 31 um das Gelenk 33 gegenüber dem langgestreckten Halter 32 abgeklappt. Bei dem Stelldraht 346 kann es sich um einen steifen Draht handeln, der auch Druckkräfte übertragen kann, um das Abschirmelement 31 zurückzuklappen. Alternativ kann eine Feder (in den Figuren nicht gezeigt) vorgesehen sein, die das Abschirmelement 31 zurückschwenkt, wenn an dem Stelldraht 346 keine Zugkraft anliegt.

Am proximalen Ende des langgestreckten Halters 32 ist, wie aus Fig. 9B ersichtlich ist, eine Griffeinrichtung vorgesehen, die dem Griff einer Schere ähnelt. Ein erstes Griffelement 341 mit einem ersten Griffring 344 ist zum Einführen des Daumens des Operateurs vorgesehen. Ein zweites Griffelement 342 mit einem zweiten Griffring 345 ist zum Einführen des Zeigefingers des Operateurs vorgesehen. Wie bei einer Schere sind das erste Griffelement 341 für den Daumen und das zweite Griffelement 342 für den Zeigefinger gegeneinander verschwenkbar. Dazu ist an dem ersten Griffelement 341 ein weiterer Bolzen 343 fest angebracht. Dieser Bolzen 343 ist von dem zweiten Griffelement 342 über eine Gleitlagerung aufgenommen. An dem zweiten Griffelement 342 ist ferner in einem Abstand von dem Bolzen 343 ein weiterer Bolzen 347 vorgesehen, an dem der Stelldraht 346 angebracht ist. Durch Auseinanderbewegen der scherenartigen Griffelemente 341 und 342 wird dadurch eine Zugbewegung auf den Stelldraht 346 ausgeübt. Diese Zugbewegung wird an dem proximalen Ende in eine Abklappbewegung des Abschirmelements 31 umgesetzt. An dem ersten Griffelement 341 sind zwei Anschläge 340 vorgesehen, die die gegenseitige Schwenkbewegung der Griffelemente 341 und 342 begrenzen. Die Anschläge 340 sind so ausgebildet, dass die Abklappbewegung des Abschirmelements 31 zwischen einer Stellung, bei der das Abschirmelement 31 und der langgestreckte Halter 32 fluchten, und einer maximalen Abklappstellung begrenzt ist.

Nun wird ein weiteres (in den Figuren nicht gezeigtes) Ausführungsbeispiel einer Betätigungsvorrichtung 34 eines Abschirmelements 31 beschrieben. Die Betätigungsvorrichtung 34 nach diesem Ausführungsbeispiel kann z. B. zur Betätigung des zuvor unter Bezug auf Fig. 5 beschriebenen Ausführungsbeispiels des Instrumentariums zum mikroinvasiven Abtragen von Spinalkanalstenosen eingesetzt werden. Die Betätigungsvorrichtung 34 nach diesem Ausführungsbeispiel ist grundsätzlich so aufgebaut wie in dem oben unter Bezug auf Fig. 9 beschriebenen Ausführungsbeispiel, zeigt jedoch einige Abweichungen und weist zusätzliche Bauteile auf. Im Unterschied zu dem Ausführungsbeispiel gemäß Fig. 9 wird über den Stelldraht 346 das Abschirmelement 31 gegenüber dem Zwischenelement 35 anstatt gegenüber dem langgestreckten Halter 32 verschwenkt, und zwar um das Gelenk 37. Zum Umlenken des Stelldrahts 346 ist bei diesem Ausführungsbeispiel anstatt der Umlenkeinrichtung 349 an dem langgestreckten Halter 32 eine andere Umlenkeinrichtung an dem Zwischenelement 35 vorgesehen. Darüber hinaus können weitere Umlenkeinrichtungen für den Stelldraht 346 vorhanden sein, z. B. an dem Zwischenelement 35 oder an dem langgestreckten Halter 32. Ferner ist ein zweiter Stelldraht vorgesehen, der an einem weiteren Bolzen angreift, der an dem Zwischenelement 35 nahe dem Gelenk 36 vorgesehen ist. Dieser zweite Stelldraht kann z. B. über ein drittes Griffelement betätigt werden, das einen dritten Griffring für den Mittelfinger aufweist.

Weitere Ausführungsbeispiele einer Betätigungsvorrichtung 34 eines Abschirmelements 31 eines erfindungsgemäßen Instrumentariums zum mikroinvasiven Abtragen von Spinalkanalstenosen werden gebildet, indem anstatt einer manuellen Betätigung eine Betätigung durch Stellglieder vorgesehen ist. Bevorzugt werden diese Stellglieder elektronisch angesteuert.

Weitere Ausführungsformen des erfindungsgemäßen Instrumentariums zum mikroinvasiven Abtragen von Spinalkanalstenosen weisen Vorrichtungen auf, mit denen sichergestellt wird, dass der Abtragekopf 21 und das Abschirmelement 31 auf dem Weg zu den Spinalkanalstenosen dorsal an dem Nervenstrang (NS) vorbeigeführt werden.

Es ist von Vorteil, wenn die Abtrageeinrichtung 2 und die Abschirmeinrichtung 3 auf dem Weg zu den Spinalkanalstenosen dorsal an dem Nervenstrang (NS) vorbeigeführt werden. In den Fig. 1 und Fig. 2 sind der bauchwärtig (ventral) gelegene Wirbelkörper (WK) und der rückwärtig (dorsal) gelegene Wirbelbogen mit Gelenkfortsätzen (GF) und Querfortsätzen (QF) zu erkennen. Dazwischen findet sich seitlich die Öffnung des Foramen Intervertebrale (FI). Durch das Foramen Intervertebrale (FI) erstreckt sich ein vom Rückenmark seitlich abzweigender Nervenstrang (NS). Bei Anwendung des erfindungsgemäßen Instrumentariums wird bevorzugt ein Zugangsweg für das Instrumentarium zum mikroinvasiven Abtragen von Spinalkanalstenosen hergestellt, der dorsal an dem Nervenstrang (NS) vorbei führt. Wird ein dorsal an dem Nervenstrang (NS) vorbeiführender Zugangsweg hergestellt, schirmt das Abschirmelement 31 den während der Anwendung überwiegend auf der dorsalen Seite freiliegenden Abtragekopf 21 nicht nur gegenüber dem Rückenmark (Dura) ab, sondern auch gegenüber dem Nervenstrang (NS). Auf diese Weise wird das Risiko einer Verletzung des Nervenstrangs (NS) vermindert.

Um zu erreichen, dass die Abtrageeinrichtung 2 und das Abschirmelement 31 dorsal an dem Nervenstrang (NS) vorbeigeführt werden, weist das Instrumentarium nach einem Ausführungsbeispiel eine Nervenwegdrängeinrichtung (in den Figuren nicht gezeigt) auf. Bevorzugt ist die Nervenwegdrangeinrichtung als langgestrecktes Instrument ausgebildet, das dazu ausgebildet ist, durch einen Arbeitskanal des Zugangsröhrchens 1 geführt zu werden. Bevorzugt weist die Nervenwegdrängeinrichtung in dem vom Operateur abgewandten Bereich eine Abschrägung (nicht gezeigt) oder ein bewegliches Element auf, das geeignet ist, eine Nervenbahn zur Seite zu drängen. Bei dem beweglichen Element kann es sich z. B. um einen schwenkbaren Arm handeln. In dem Zugangsröhrchen 1 ist zur Durchführung der Nervenwegdrängeinrichtung ein von dem ersten Arbeitskanal 12 und ggf. dem zweiten Arbeitskanal für die Optik und ggf. dem dritten Arbeitskanal für ein separat von der Abtrageeinrichtung 2 durchgeführtes Abschirmelement 31 verschiedener vierter Arbeitskanal vorgesehen. Das Zugangsröhrchen 1 kann dann insgesamt fünf oder mehr Kanäle aufweisen, z. B. den ersten Arbeitskanal 12, den zweiten Arbeitskanal zum Durchführen einer Optik, den vierten Arbeitskanal und zwei Spülkanäle oder sogar sechs oder mehr Kanäle aufweisen, z. B. den ersten Arbeitskanal 12, den zweiten Arbeitskanal zum Durchführen einer Optik, den dritten Arbeitskanal zum Durchführen des Abschirmelements 31, den vierten Arbeitskanal und zwei Spülkanäle.

Weitere Ausführungsbeispiele des erfindungsgemäßen Instrumentariums können ferner ein Zugangsweginstrumentarium (in den Figuren nicht gezeigt) aufweisen, um zu erreichen, dass ein dorsal an dem Nervenstrang (NS) vorbeiführender Zugangsweg hergestellt wird. Das Zugangsweginstrumentarium kann vorzugsweise ein als Operationsnadel, vorzugsweise als Spinalnadel, ausgeführtes nadelförmiges Zugangsweginstrument aufweisen. Das nadelförmige Zugangsweginstrument wird vom seitlichen Rücken ausgehend in Richtung zu dem Foramen Intervertebrale in den Körper eingeführt. Das nadelförmige Zugangsweginstrument wird eingeführt, bis die Spitze in dem Bereich des Foramen Intervertebrale angelangt ist. Anschließend werden weitere zu dem Zugangsweginstrumentarium gehörende Zugangsweginstrumente eingesetzt, um den geschaffenen Zugangsweg aufzuweiten. Diese weiteren Zugangsweginstrumente sind als Dilatationsröhrchen ausgebildet, die auf das nadelförmige Zugangsweginstrument und ggf. auf bereits vorher aufgeschobene Dilatationsröhrchen aufgeschoben werden. Jedes Dilatationsröhrchen weist einen größeren Außendurchmesser auf als das nadelförmige Zugangsweginstrument und als sämtliche zuvor bereits aufgeschobenen Dilatationsröhrchen und weist einen Innendurchmesser auf, der geeignet ist, die zuvor aufgeschobenen Elemente aufzunehmen. Die Dilatationsröhrchen können an der Stirnseite ein fräserartiges Profil aufweisen, um bei rotierender Betätigung ein Zerteilen des durchstoßenen Gewebes zu fördern.

Der Zugangsweg für das erfindungsgemäße Instrumentarium wird bevorzugt so gewählt, dass er in einer zur Körperlängsrichtung senkrechten Ebene gegenüber der dorsalen Richtung um einen Winkel von mehr als 70°, weiter bevorzugt mehr als 80° abgewinkelt verläuft. Das nadelförmige Zugangsweginstrument, die Nervenwegdrängeinrichtung, die Abtrageeinrichtung 2 und/oder die Abschirmeinrichtung 3 werden daher besonders weit außen am Rücken eingeführt. Das nadelförmige Zugangsweginstrument hat zu diesem Zweck eine Länge von mehr als 15 cm, bevorzugt mehr als 20 cm, weiter bevorzugt mehr als 25 cm. Die Abtrageeinrichtung 2 hat zu diesem Zweck eine Länge von mehr als 20 cm, bevorzugt mehr als 25 cm, weiter bevorzugt mehr als 30 cm. Die Abschirmeinrichtung 3 hat zu diesem Zweck eine Länge von mehr als 20 cm, bevorzugt mehr als 25 cm, weiter bevorzugt mehr als 30 cm.

Bevorzugt weist das nadelförmige Zugangsweginstrument im Bereich seiner Spitze eine Sensoranordnung auf, die eine physikalische Größe oder mehrere physikalische Größen aufnimmt, die geeignet ist bzw. sind, die Nähe und/oder die relative Lage von Nervenbahnen zu bestimmen. Mittels einer Auswerteeinheit werden die über die Sensoranordnung aufgenommenen Signale ausgewertet und die relative Lage zwischen dem Zugangsweginstrument und den Nervenbahnen wird ermittelt. Diese relative Lage kann an einem Bildschirm angezeigt werden. Anhand dieser Anzeige kann der Operateur feststellen, ob das nadelförmige Zugangsweginstrument, das den Zugangsweg bestimmt, an der dorsalen oder an der ventralen Seite des Nervenstrangs (NS) positioniert ist. Nimmt das Zugangsweginstrument eine Stellung an der nicht gewünschten Seite des Nervenstrangs (NS) ein, kann der Operateur das nadelförmige Zugangsweginstrument herausziehen und erneut einführen, um die gewünschte Lage zu erreichen. Anhand der Anzeige kann der Operateur außerdem sicherstellen, dass das Zugangsweginstrument den Nervenstrang (NS) nicht verletzt.

Weiter kann das erfindungsgemäße Instrumentarium zum mikroinvasiven Abtragen von Spinalkanalstenosen ein Bildgebungsgerät aufweisen, mit dem die Lage des Zugangsweginstruments, der Nervenwegdrängeinrichtung, der Abtrageeinrichtung 2 und/oder der Abschirmeinrichtung 3 aufgenommen wird. Das Bildgebungsgerät kann z. B. die Verfahren der Computertomographie (CT), Magnetresonanztomographie (MRT), Szintigrafie oder nativen Röntgenaufnahme nutzen. Wenn ein Verfahren genutzt wird, das nicht geeignet ist, die Lage des Nervenstranges (NS) aufzunehmen, wie z. B. die Computertomographie, so wird eine Auswerteeinrichtung genutzt, um die relative Lage zwischen dem Zugangsweginstrument, der Nervenwegdrängeinrichtung, der Abtrageeinrichtung und/oder der Abschirmeinrichtung und dem Nervenstrang (NS) zu ermitteln. Die Auswerteeinrichtung verfügt über einen Speicher, in dem Daten über die individuellen anatomischen bzw. geometrischen Verhältnisse an der Operationsstelle bei dem zu behandelnden Patienten gespeichert sind. Die Daten können zuvor z. B. über Magnetresonanztomographie (MRT) aufgenommen worden sein. Aus diesen Daten ermittelt die Auswerteeinrichtung eine Darstellung des Nervenstrangs (NS), die im Hinblick auf Blickwinkel, Maßstab und andere Darstellungsparameter der mit dem Bildgebungsgerät erstellten Aufnahme der aktuellen Position und Lage des Zugangsweginstruments, der Nervenwegdrängeinrichtung, der Abtrageeinrichtung 2 und/oder der Abschirmeinrichtung 3 entsprechen. Die Aufnahme des jeweiligen Instruments und die Darstellung des Nervenstrangs (NS) werden dann einander überlagert auf einem Bildschirm ausgegeben, so dass der Operateur unter der Annahme, dass sich der Nervenstrang (NS) nicht verlagert hat, die relative Lage zwischen Zugangsweginstrument, Nervenwegdrangeinrichtung, Abtrageeinrichtung 2 und/oder Abschirmeinrichtung einerseits und Nervenstrang (NS) andererseits ersehen kann. Die Auswerteeinrichtung kann durch eine geeignete Kombination von Computerhardware und -software realisiert werden.

Bei weiteren Ausführungsformen ist das erfindungsgemäße Instrumentarium zum mikroinvasiven Abtragen von Spinalkanalstenosen mit einer Steuereinrichtung ausgestattet (in den Figuren nicht gezeigt). Wenn das Instrumentarium Betätigungsvorrichtungen mit Stellantrieben aufweist, kann die Steuereinrichtung die Stellantriebe ansteuern, indem sie an diese Steueranweisungen übermittelt. Dies kann mittels leitungsgebundener oder drahtloser Übertragungstechnik erfolgen. Wenn das Instrumentarium manuelle Betätigungsvorrichtungen aufweist, werden die von der Steuereinrichtung erarbeiteten Steueranweisungen über eine akustische oder eine visuelle Ausgabeeinrichtung, wie z. B. eine Ansageeinrichtung oder einen Monitor an den Operateur ausgegeben. Eine Steuereinrichtung für das oben unter Bezug auf Fig. 5 beschriebene Instrumentarium, das den langgestreckten Halter 32, das Abschirmelement 31 und das Zwischenelement 35 aufweist, kann z. B. Steueranweisungen abgeben zum:
- Vorschieben des Zugangsröhrchens 1 in das Foramen Intervertebrale bzw. zum Zurückziehen aus dem Foramen Intervertebrale;
- Vorschieben des Abschirmelements 31 aus dem distalen Ende 11 des Zugangsröhrchens 1 heraus bzw. Zurückziehen des Abschirmelements 31 in das Zugangsröhrchen 1 hinein;
- Abklappen bzw. Zurückklappen des Abschirmelements 31 gegenüber dem Zwischenelement 35;
- Abklappen bzw. Zurückklappen des Zwischenelements 35 gegenüber dem langgestreckten Halter 32.

Die von der Steuereinrichtung abgegebenen Steueranweisungen können auf einem vorprogrammierten Verfahrprogramm beruhen. Bevorzugt werden bei der Ermittlung der Steueranweisungen Daten über die individuellen Verhältnisse bei dem Patienten berücksichtigt, wie z. B. die genaue Lage und Form der Spinalkanalstenose, aber auch die genauen Abmessungen des Wirbelkörpers. Diese den Patienten betreffenden Daten können mittels eines üblichen Bildgebungsverfahrens, z. B. der Computertomographie gewonnen werden. Weiter bevorzugt werden auch die genaue Größe und Gestalt des Instrumentariums berücksichtigt. Weiter bevorzugt werden während des Operationsablaufs die aktuelle Position des Instrumentariums und die aktuelle Lage und Form des Wirbelkörpers und der Spinalkanalstenose aufgenommen und wird der Programmablauf darauf angepasst.

Es wird nun eine Betätigungs- und Steuereinrichtung des erfindungsgemäßen Instrumentariums zum mikroinvasiven Abtragen von Spinalkanalstenosen nach einer Ausführungsform der Erfindung beschrieben. Diese Betätigungs- und Steuereinrichtung übernimmt ganz oder teilweise den Antrieb der verschiedenen Bewegungen des Instrumentariums und/oder deren Steuerung. Die Betätigungs- und Steuereinrichtung weist einen Prozessor, einen Datenspeicher, einen Bildschirm sowie ein erstes, ein zweites, ein drittes, ein viertes und ein fünftes Stellglied auf. Das erste Stellglied treibt eine Vor- und Rückzugsbewegung des Zugangsröhrchens 1 an. Das zweite Stellglied treibt eine Vor- und Rückzugsbewegung des langgestreckten Halters 32 an. Das dritte Stellglied treibt eine Abklappbewegung des Gelenks 36 zwischen dem langgestreckten Halter 32 und dem Zwischenelement 35 an. Das vierte Stellglied treibt eine Abklappbewegung des Gelenks 37 zwischen dem Zwischenelement 35 und dem Abschirmelement 31 an. Das fünfte Stellglied treibt eine Rotationsbewegung des bei diesem Ausführungsbeispiel als Fräserkopf ausgestalteten Abtragekopfs 21 an.

Der Datenspeicher umfasst einen Physiodatenspeicher, in dem die Geometrien und Abmessungen der relevanten Bestandteile des Körpers des Patienten in der Operationszone gespeichert sind, vorzugsweise in Form von hoch aufgelösten 3D-Daten wie bei einem CAD-System. Diese Daten können durch herkömmliche Bildgebungsverfahren wie z. B. Computertomographie (CT) oder Magnetresonanztomographie (MRT), Szintigrafie, native Röntgenaufnahme oder ähnliches etc. ermittelt worden sein. Ferner können in dem Physiodatenspeicher weitere physiologische Daten gespeichert sein wie z. B. Daten über den Grad der Verhärtung einer Verknöcherung, die Dichte bestimmter Zonen etc. Ferner umfasst der Datenspeicher einen Instrumentendatenspeicher, in dem die Geometrien und Abmessungen des verwendeten Operationsinstrumentariums gespeichert sind. Auch diese Daten liegen vorzugsweise in Form von hoch aufgelösten 3D-Daten wie bei einem CAD-System vor. Ferner umfasst der Datenspeicher einen Instrumenten-Positions-Speicher, in dem die aktuellen Positionen und Stellungen des verwendeten Operationsinstrumentariums, geordnet nach den einzelnen Bestandteilen des Instrumentariums gespeichert sind. Weiter umfasst der Datenspeicher einen Stellgliederdatenspeicher, in dem die Leistungsdaten der Stellglieder des verwendeten Operationsinstrumentariums gespeichert sind. Darüber hinaus umfasst der Datenspeicher einen Operationsprogrammspeicher, in dem Steuerprogramme für bestimmte Bewegungsabläufe gespeichert sind, die die Bestandteile des Instrumentariums im Zuge der Operation ausführen. Dabei handelt es sich bevorzugt um Programme, die bei der Gestaltung der Bewegungsabläufe Daten aus dem Physiodatenspeicher, Instrumentendatenspeicher, dem Instrumenten-Positions-Speicher und/oder dem Stellgliederdatenspeicher berücksichtigen.

Weiter bevorzugt weist die Betätigungs- und Steuereinrichtung ein oder mehrere Programme zur Operationssteuerung durch den Arzt auf. So wird auf dem Bildschirm bevorzugt eine Eingabemaske ausgegeben, über die der Bediener, z. B. der Arzt, Operationsprogramme auswählen und/oder einzelne Operationsschritte festlegen kann, die sodann mittels des Instrumentariums einschließlich der Betätigungs- und Steuereinrichtung ausgeführt werden. Über den Bildschirm kann auch mittels eines Bildgebungsverfahrens die aktuelle Position und Stellung des Instrumentariums angezeigt werden.

Erfindungsgemäß ist es möglich, dass die Betätigungs- und Steuereinrichtung einzelne oder einige der zuvor erwähnten Bewegungen und Abläufe steuert und antreibt und andere Abläufe und Arbeitsschritte manuell vorgenommen werden. Dabei ist es z. B. möglich, dass das Steuerprogramm den automatisch gesteuerten und angetriebenen Ablauf abarbeitet und dem Operateur dann an geeigneter Stelle anzeigt, dass nun bestimmte manuelle Arbeiten anstehen.

Das erfindungsgemäß gestaltete Instrumentarium kann außer zum mikroinvasiven Abtragen von Spinalkanalstenosen auch für andere mikroinvasive Operationen an der Wirbelsäule oder an anderen Körperteilen eingesetzt werden. Bevorzugt wird es zum mikroinvasiven Abtragen von Spinalkanalstenosen, insbesondere am Ligamentum Flavum eingesetzt.

## Patentansprüche

1. Instrumentarium zum mikroinvasiven Behandeln von Spinalkanalstenosen und/oder anderen Einengungen des Spinalkanals, aufweisend
- eine Abtrageeinrichtung (2) mit einem Abtragekopf (21), der von einem mikroinvasiven Zugangsröhrchen (1), das ein in den Körper einführbares distales Ende aufweist, aufnehmbar ist und der an dem distalen Ende aus dem Zugangsröhrchen (1) herausführbar und in mindestens eine Arbeitsposition bringbar ist;
- ein Abschirmelement (31) zum Abschirmen des Abtragekopfes (21) gegenüber der Dura, das von dem mikroinvasiven Zugangsröhrchen (1) aufnehmbar ist und das an dem distalen Ende aus dem Zugangsröhrchen (1) herausführbar und in mindestens eine Arbeitsstellung bringbar ist, in der das Abschirmelement (31) das Zugangsröhrchen (1) lateral überragt;
- einen langgestreckten Halter (32), an dem das Abschirmelement (31) mittelbar abgestützt ist, wobei der langgestreckte Halter (32) und das Abschirmelement (31) in einer ersten Stellung des Abschirmelements (31) im Wesentlichen zueinander fluchtend angeordnet sind und das Abschirmelement (31) in der Arbeitsstellung gegenüber dem langgestreckten Halter (32) abgeklappt angeordnet ist; und
- zwischen dem langgestreckten Halter (32) und dem Abschirmelement (31) mindestens ein Zwischenelement (35), das gegenüber dem langgestreckten Halter (32) abklappbar ist und an dem das Abschirmelement (31) abklappbar angeordnet ist, **gekennzeichnet durch**
- eine Betätigungsvorrichtung (34), mittels der das Abschirmelement (31) gegenüber dem Zwischenelement (35) abgeklappt werden kann, während der langgestreckte Halter (32) und das Zwischenelement (35) zueinander fluchtend verbleiben.

2. Instrumentarium nach Anspruch 1, wobei der Abtragekopf (21) an der Seite des Abschirmelements (31) angeordnet wird, zu der hin das Abschirmelement (31) abgeklappt wird.

3. Instrumentarium nach einem der vorangehenden Ansprüche, wobei die Abtrageeinrichtung (2) an dem Abschirmelement (31) gelagert ist.

4. Instrumentarium nach einem der vorangehenden Ansprüche, wobei das Abschirmelement (31) von seinem proximalen Ende zum distalen Ende einen gekrümmten Verlauf aufweist.

5. Instrumentarium nach einem der vorangehenden Ansprüche, wobei das Abschirmelement (31) von seinem proximalen Ende zum distalen Ende einen S-förmig gekrümmten Verlauf aufweist derart, dass das distale Ende in Abklapprichtung gekrümmt ist und sich zu dem proximalen Ende hin ein entgegengesetzt gekrümmter längerer Bogen anschließt.

6. Instrumentarium nach einem der vorangehenden Ansprüche, wobei das Abschirmelement (31) den Abtragekopf (21) weitgehend umgibt und an der Seite, zu der hin das Abschirmelement (31) bewegt wird, wenn es aus dem Zugangsröhrchen (1) herausgeführt und in die Arbeitsstellung gebracht wird, mit einer fensterartigen Aussparung (314) versehen ist, in der der Abtragekopf (21) freiliegt.

7. Instrumentarium nach einem der vorangehenden Ansprüche, wobei der Abtragekopf (21) als Fräserkopf ausgebildet ist und einen Fräserabschnitt (211) aufweist, der zu dem proximalen Ende hin durch einen proximalen Fräserabsatz (213) begrenzt ist, an dem sich der Durchmesser des Abtragekopfs (21) um mindestens 0,5 mm, bevorzugt um mindestens 1 mm, weiter bevorzugt um mindestens 2 mm, verringert, und an den sich ein proximaler Freiraumabschnitt (214) des Abtragekopfes (21) anschließt, der in der fensterartigen Aussparung (314) freiliegt.

8. Instrumentarium nach einem der vorangehenden Ansprüche, wobei der proximale Freiraumabschnitt (214) eine Länge (h2) von mindestens 2 mm, bevorzugt von mindestens 4 mm, weiter bevorzugt von mindestens 6 mm aufweist.

9. Instrumentarium nach einem der vorangehenden Ansprüche, wobei der Abtragekopf (21) in seiner Position an dem Abschirmelement (31) verstellbar ist, insbesondere in Längsrichtung und/oder in Querrichtung des Abschirmelements (31).

10. Instrumentarium nach einem der vorangehenden Ansprüche, wobei der Abtragekopf (21) über den distalen Rand und/oder über einen seitlichen Rand des Abschirmelements (31) hinaus verstellbar ist.

11. Instrumentarium nach einem der vorangehenden Ansprüche, wobei die Abtrageeinrichtung (2) eine Welle (22) mit mindestens einem flexiblen Abschnitt oder einem Kardangelenk aufweist.

12. Instrumentarium nach einem der vorangehenden Ansprüche, wobei die Welle (22) an dem Abschirmelement (31) mittels mehrerer Gleitlager oder Kugellager gelagert ist.

13. Instrumentarium nach einem der vorangehenden Ansprüche, wobei das Instrumentarium eine endoskopische Optik aufweist.

14. Instrumentarium nach einem der vorangehenden Ansprüche, wobei das Instrumentarium das Zugangsröhrchen (1) aufweist, und dieses an seinem distalen Ende solche Außenabmessungen aufweist, dass es durch das Foramen Intervertebrale in den Zwischenraum zwischen den Wirbelkörpern einführbar ist, insbesondere einen kreisförmigen Außenquerschnitt von weniger als 12 mm.

15. Instrumentarium nach einem der vorangehenden Ansprüche, wobei das Zugangsröhrchen (1) mindestens fünf Kanäle aufweist, aufweisend zwei Arbeitskanäle, einen Kanal zum Durchführen einer endoskopischen Optik und zwei Spülkanäle.

## Claims

1. An instrument set for the microinvasive treatment of stenoses of the spinal canal and/or other constrictions of the spinal canal, comprising
- an ablation device (2) having an ablation head (21) which can be accommodated in a microinvasive access tube (1), which has a distal end insertable into the body and which can be directed out of the access tube (1) at the distal end and brought into at least one working position;
- a screening element (31) for screening the ablation head (21) in relation to the dura which can be accommodated in the microinvasive access tube (1) and which can be directed out of the access tube (1) at the distal end and brought into at least one working position in which the screening element (31) laterally protrudes above the access tube (1);
- an elongated mount (32) on which the screening element (31) is indirectly supported, wherein the elongated mount (32) and the screening element (31) are arranged so as to be substantially aligned with respect to each other in a first position of the screening element (31) and the screening element (31) is arranged so that it is pivoted with respect to the elongated mount (32) in the working position; and
- at least one intermediate element (35), which can be pivoted with respect to the elongated mount (32) and on which the screening element (31) is pivotably disposed, arranged between the elongated mount (32) and the screening element (31),
**characterized by**
- a control mechanism (34) with the aid of which the screening element (31) can be pivoted in relation to the intermediate element (35), while the elongated mount (32) and the intermediate element (35) remain aligned towards each other.

2. The instrument set according to claim 1, wherein the ablation head (21) is positioned at the side of the screening element (31) towards which the screening element (31) is pivoted.

3. The instrument set according to one of the preceding claims, wherein the ablation device (2) is supported on the screening element (31).

4. The instrument set according to one of the preceding claims, wherein the screening element (31) has a curved progression from its proximal end to its distal end.

5. The instrument set according to one of the preceding claims, wherein the screening element (31) has an S-shaped, curved progression from its proximal end to its distal end so that the distal end is curved in the pivoting direction and that a longer arc curved in the opposite direction follows towards the proximal end.

6. The instrument set according to one of the preceding claims, wherein the screening element (31) surrounds the ablation head (21) to a large extent and is provided with a window-like recess (314) in which the ablation head (21) is exposed at the side towards which the screening element (31) is moved when it is directed out of the access tube (1) and brought into the working position.

7. The instrument set according to one of the preceding claims, wherein the ablation head (21) is formed as a cutting head and comprises a cutting section (211) which is confined by a proximal cutter ledge (213) at which the diameter of the ablation head (21) decreases by at least 0.5 mm, preferably by at least 1 mm, more preferably by at least 2 mm towards the proximal end and which is followed by a proximal clearance section (214) of the ablation head (21) which is exposed in the window-like recess (314).

8. The instrument set according to one of the preceding claims, wherein the proximal clearance section (214) has a length (h2) of at least 2 mm, preferably of at least 4 mm, more preferably of at least 6 mm.

9. The instrument set according to one of the preceding claims, wherein the position of the ablation head (21) on the screening element (31) is adjustable, particularly in the longitudinal direction and/or in the transverse direction of the screening element (31).

10. The instrument set according to one of the preceding claims, wherein the ablation head (21) is adjustable beyond the distal edge and/or a lateral edge of the screening element (31).

11. The instrument set according to one of the preceding claims, wherein the ablation device (2) comprises a shaft (22) having at least a flexible section or a universal joint.

12. The instrument set according to one of the preceding claims, wherein the shaft (22) is supported on the screening element (31) by means of several plain or ball bearings.

13. The instrument set according to one of the preceding claims, wherein the instrument set comprises endoscopic optics.

14. The instrument set according to one of the preceding claims, wherein the instrument set exhibits the access tube (1) which has such exterior dimensions at its distal end that it is insertable into the clearance between the vertebral bodies through the foramen intervertebrale, especially a circular cross section of less than 12 mm.

15. The instrument set according to one of the preceding claims, wherein the access tube (1) comprises at least five passages, namely two working passages, a passage for passing through endoscopic optics, and two rinsing passages.

## Revendications

1. Instrument de traitement microinvasif des sténoses du canal rachidien et/ou les autres rétrécissements du canal rachidien, présentant
- un dispositif d'ablation (2) avec une tête d'ablation (21) pouvant être fixée à un tube d'accès microinvasif (1) possédant une extrémité distale introductible dans le corps et sortie du tube d'accès (1) par l'extrémité distale et amenée dans au moins une position de travail ;
- un élément de protection (31) pour protéger la tête d'ablation (21) contre la dure-mère, pouvant être fixé sur le tube d'accès microinvasif (1) et sorti du tube d'accès (1) par l'extrémité distale et amené dans au moins une position de travail, dans laquelle l'élément de protection (31) dépasse latéralement du tube d'accès (1) ;
- un support allongé (32) sur lequel l'élément de protection (31) est directement appuyé, le support allongé (32) et l'élément de protection (31) étant relativement alignés lorsque l'élément de protection (31) est dans une première position, l'élément de protection (31) étant rabattu contre le support allongé (32) lorsqu'il est en position de travail ; et
- entre le support allongé (32) et l'élément de protection (31) se trouve au moins un élément intermédiaire (35) rabattable par rapport au support allongé (32) et contre lequel l'élément de protection (31) est rabattu, **caractérisé en ce que**
- un dispositif d'actionnement (34) permettant de rabattre l'élément de protection (31) contre l'élément intermédiaire (35), tandis que le support allongé (32) et l'élément intermédiaire (35) restent alignés.

2. Instrument d'après la revendication 1, la tête d'ablation (21) étant disposée sur le côté de l'élément de protection (31) vers lequel l'élément de protection (31) est rabattu.

3. Instrument d'après l'une des revendications ci-dessus, le dispositif d'ablation (2) reposant sur l'élément de protection (31).

4. Instrument d'après l'une des revendications ci-dessus, l'élément de protection (31) présentant un corps courbé de son extrémité proximale à son extrémité distale.

5. Instrument d'après l'une des revendications ci-dessus, l'élément de protection (31) formant une courbe en forme de S de son extrémité proximale à son extrémité distale, de sorte que l'extrémité distale est courbée direction rabattement, et se connecte à un arc plus long de courbure inverse au niveau de l'extrémité proximale.

6. Instrument d'après l'une des revendications ci-dessus, l'élément de protection (31) entourant la majeure partie de la tête d'ablation (21) et étant doté d'une fenêtre (314) dans laquelle la tête d'ablation (21) repose librement sur le côté vers lequel l'élément de protection (31) est déplacé lorsqu'il est sorti du tube d'accès (1) et amené en position de travail.

7. Instrument d'après l'une des revendications ci-dessus, la tête d'ablation (21) prenant la forme d'une tête fraiseuse présentant une section de fraiseuse (211) limitée à l'extrémité proximale par un talon de fraiseuse proximal (213), sur lequel le diamètre de la tête d'ablation (21) est réduit d'au moins 0,5 mm, ou mieux d'au moins d'1 mm, ou dans l'idéal d'au moins 2 mm, et auquel est connecté une section libre (214) de la tête d'ablation (21) qui repose librement sur la fenêtre (314).

8. Instrument d'après l'une des revendications ci-dessus, la section libre proximale (214) présentant une longueur (h2) d'au moins 2 mm, ou mieux d'au moins 4 mm et dans l'idéal d'au moins 6 mm.

9. Instrument d'après l'une des revendications ci-dessus, la position de la tête d'ablation (21) sur l'élément de protection (31) étant réglable, en particulier en direction longitudinale et/ou transversale de l'élément de protection (31).

10. Instrument d'après l'une des revendications ci-dessus, la tête d'ablation (21) étant réglable au-delà de la bordure distale et/ou d'une bordure latérale de l'élément de protection (31).

11. Instrument d'après l'une des revendications ci-dessus, le dispositif d'ablation (2) présentant un arbre (22) doté au moins d'une section flexible ou d'un cardan.

12. Instrument d'après l'une des revendications ci-dessus, l'arbre (22) reposant sur l'élément de protection (31) à l'aide de plusieurs paliers ou roulements.

13. Instrument d'après une des revendications ci-dessus, l'instrument présentant l'aspect d'un endoscope.

14. Instrument d'après l'une des revendications ci-dessus, l'instrument présentant un tube d'accès (1) affichant à son extrémité distale des dimensions extérieures lui permettant d'être introduit à travers le foramen intervertébral dans l'espace intermédiaire entre les corps vertébraux, et notamment une coupe transversale extérieure circulaire de moins de 12 mm.

15. Instrument d'après l'une des revendications ci-dessus, la tube d'accès (1) présentant au moins cinq canaux, soit deux canaux de travail, un canal d'introduction d'endoscope et deux canaux de rinçage.
